# EUROPÄISCHE PATENTSCHRIFT

(11) **EP 3 458 304 B1**
(45) Veröffentlichungstag und Bekanntmachung des Hinweises auf die Patenterteilung: **15.07.2020**
(21) Anmeldenummer: 17723315.2
(22) Anmeldetag: 03.05.2017
(51) Int. Cl.: B60N 2/00, B60N 2/75

(54) **SITZBELEGUNGSERKENNUNG**
SEAT OCCUPANCY RECOGNITION
RECONNAISSANCE D'OCCUPATION DE SIÈGE

(30) Priorität: 17.05.2016 DE 102016109008; 17.05.2016 DE 102016109013; 31.03.2017 DE 102017106949
(43) Veröffentlichungstag der Anmeldung: 27.03.2019
(73) Patentinhaber: GRAMMER AG, 92224 Amberg (DE)
(72) Erfinder: ÜBELACKER, Roland, 92536 Pfreimd (DE); KOLB, Jens, 92281 Königstein (DE); KULLMANN, Achim, 92224 Amberg (DE)
(74) Vertreter: Hannke, Christian
(86) Internationale Anmeldenummer: PCT/EP2017/060463
(87) Internationale Veröffentlichungsnummer: WO 2017/198459

(56) Entgegenhaltungen:
- US-A1- 2007 120 347
- US-A1- 2007 135 982

## Beschreibung

Die Erfindung betrifft eine Sitzbelegungserkennung für einen Fahrzeugsitz, insbesondere Nutzfahrzeugsitze und ein Verfahren zum Erkennen einer Sitzbelegung.

Aus dem Stand der Technik sind Fahrzeugsitze bekannt, mittels welcher erkennbar ist, ob ein Fahrzeugsitz belegt ist oder nicht. Einfache Sitze mit einer Sitzbelegungserkennung verwenden Sensordaten eines Sensors, mittels welchem ein Gewicht auf dem Fahrzeugsitz erkennbar ist und schließen dann rein daraus, dass der Sitz mit einem Menschen belegt ist. Aus der US 2007/120347 ist ein Verfahren zum Erkennen einer Sitzbelegung eines Sitzes bekannt welches folgende Verfahrensschritte aufweist:
a. Durchführen einer Erkennung einer Form eines Gegenstands
b. Durchführen einer Körperfunktionserfassung mittels einer Körperfunktionserfassungseinrichtung
c. Durchführen einer Körperfunktionserkennung mittels einer Körperfunktionserkennungseinrichtung nach positiver Körperfunktionserfassung.

Dieses Verfahren bzw. der Fahrzeugsitz gemäß dem Stand der Technik ist aber nicht in der Lage, genau zu erkennen, ob sich nun ein Mensch, ein Tier oder ein beliebiger Gegenstand, beispielsweise eine Bierkiste, auf dem Nutzfahrzeugsitz befindet.

Es ist daher Aufgabe der vorliegenden Erfindung, eine Sitzbelegungserkennung sowie einen Fahrzeugsitz, insbesondere einen Nutzfahrzeugsitz, mit einer Sitzbelegungserkennung und ein Verfahren bereitzustellen, mittels welchen erkennbar ist, ob ein Mensch auf einem Fahrzeugsitz sitzt und diesen belegt.

Kerngedanke dieser Erfindung ist es, ein Verfahren zum Erkennen einer Sitzbelegung eines Sitzes nach Anspruch 1 bereitzustellen.

Das Verfahren zum Erkennen einer Sitzbelegung eines Sitzes, insbesondere eines Nutzfahrzeugsitzes, umfasst dabei erfindungsgemäß einen ersten Verfahrensschritt einer Volumendetektion mittels einer Volumendetektionseinrichtung.

Unter einem Volumen ist zumindest ein Teilvolumen eines menschlichen Körpers zu verstehen. Mittels einer Volumendetektionseinrichtung kann daher erkannt werden, dass zumindest das Teilvolumen des menschlichen Körpers sich auf dem Fahrzeugsitz befindet. Ein Volumen kann hierbei auch durch eine Massendetektion und/oder einer Umrissdetektion erkannt werden, da auch durch derartige Detektionsmethoden auf ein Volumen geschlossen werden kann.

Vorteilhafterweise umfasst die Volumendetektionseinrichtung mindestens einen Sensor, welcher zur Detektion eines Volumens vorgesehen und ausgebildet ist, um ein Volumen, beispielsweise auch durch Umrisse oder eine Masse, detektiert werden können. Ein derartiger Sensor kann als ein kapazitiver Sensor, ein induktiver Sensor, eine Lichtschranke, ein elektromagnetischer Sensor oder ein Ultraschallsensor oder eine Kombination daraus ausgebildet sein. Bevorzugt handelt es sich hierbei um einen kapazitiven Sensor, durch welchen ein elektrisches Feld erzeugt werden kann. Wird dabei in den Sensorbereich des Sensors ein Volumen eingebracht oder befindet sich dort ein Volumen, so ändert sich der Wert des zu dem elektrischen Feld gehörende elektrische Flussdichte aufgrund der Änderung der dielektrischen Leitfähigkeit durch das Volumen.

Mittels einer Volumendetektion durch eine Volumendetektionseinrichtung ist jedoch noch nicht unterscheidbar, ob sich ein Mensch oder ein Gegenstand auf dem Fahrzeugsitz befindet. Es ist daher nötig, dass das Verfahren weitere Verfahrensschritte umfasst, mittels welchen entschieden werden kann, ob sich ein Mensch oder ein nicht-lebendiger Gegenstand auf dem Fahrzeugsitz befindet.

Weiter vorteilhaft umfasst das Verfahren daher erfindungsgemäß einen zweiten Verfahrensschritt zum Erfassen von Körperfunktionen mittels einer Körperfunktionenerfassungseinrichtung.

Unter Körperfunktionen ist hierbei besonders vorteilhaft eine Herzfunktion und/oder eine Lungenfunktion zu verstehen. So kann hierbei mittels der Körperfunktionenerfassungseinrichtung insbesondere entsprechend ein Herzschlag mit einer Herzfrequenz und/oder ein Puls oder eine Atmungsfrequenz detektiert werden. Ebenso kann eine Bewegung, beispielsweise von Körperteilen, des Volumens hierzu erkannt werden.

Weiter können beispielsweise andere Körperfunktionen wie zum Beispiel eine Temperatur des Fahrzeugführers, die Masse des Fahrzeugführers, der Sauerstoffgehalt des Blutes des Fahrzeugführers, eine Transpiration des Fahrzeugführers, eine Körperkontur, die Bewegung des Körpers, die Magenakustik, Hirnwellen, Muskelsignale, Hautwiderstand, Körpergeruch und dergleichen detektiert werden.

Die Körperfunktionenerfassungseinrichtung umfasst dabei abhängig von den zu erfassenden Körperfunktionen entsprechende Sensoren, die optisch, elektrisch, magnetisch, elektromagnetisch, thermisch, mechanisch und andere Sensordaten aufnehmen können.

Erfindungsgemäss umfasst das Verfahren einen dritten Verfahrensschritt einer Durchführung einer Körperfunktionserkennung mittels einer Körperfunktionenerkennungseinrichtung nach positiver Körperfunktionserfassung. Mittels dieses Verfahrensschrittes wird überprüft, ob die mittels der Körperfunktionenerfassungseinrichtung aufgenommenen Sensordaten einem menschlichen Körper zuordenbar sind. Hierdurch kann ausgeschlossen werden, dass sich ein Tier auf dem Fahrzeugsitz befindet, sondern tatsächlich ein menschliches Wesen. Unter positiver Körperfunktionserfassung ist also hierbei zu verstehen, dass Körperfunktionen erfasst worden sind. Bevorzugt handelt es sich hierbei um Biosignale und/oder Körperbewegungen, und vorzugsweise handelt es sich bei den Körperfunktionen daher um eine Herzfunktion und/oder eine Lungenfunktion als Beispiele für Biosignale, da diese besonders charakteristisch für die bestimmten Lebewesen sind. Mittels der Körperfunktionserfassung kann erkannt werden, ob sich ein Lebewesen auf dem Fahrzeugsitz befindet, insbesondere kann erkannt werden, ob sich ein Mensch auf dem Fahrzeugsitz befindet. Zur Unterscheidung werden die Biosignale und/oder Körperbewegungen herangezogen.

Verläuft eine Sitzbelegungserkennung positiv, also wurde erkannt, dass sich ein Mensch auf dem Fahrzeugsitz befindet, so können die durch die Körperfunktionenerfassungseinrichtung aufgenommenen Sensordaten für weitere Funktionen, welche über die Sitzbelegungserkennung hinausgehen, verwendet werden.

Gemäß einer weiteren bevorzugten Ausführungsform wird nach einer negativen Körperfunktionserkennung entweder die Körperfunktionserfassung erneut durchgeführt, oder mit dem Verfahrensschritt a. fortgefahren, oder zusätzliche Körperfunktionen durch die Körperfunktionserfassungseinrichtung erfasst. Das bedeutet also, dass entweder Verfahrensschritt b. nochmals durchgeführt wird, oder die Verfahrensschritte a. und b., oder weitere Körperfunktionen erfasst werden, welche zur Entscheidungsfindung herangezogen werden können, ob es sich bei dem erkannten Volumen auf dem Fahrzeugsitz um einen Menschen handelt oder nicht.

Gemäß einer weiteren Ausführungsform ist die zusätzliche Körperfunktion zumindest eine ausgewählt aus einer Temperatur, einer Transpiration, einer Magenakustik, Körpergeruch und Hirnwellen und/oder eine Kombination davon.

So ist es etwa denkbar, dass zumindest ein Teil der aufgenommenen Sensordaten der Sitzbelegungserkennung, insbesondere der Körperfunktionenerfassungseinrichtung, dem Fahrzeugführer mittels eines Displays oder dergleichen angezeigt werden. So kann der Fahrzeugführer beispielsweise seine aktuelle Herzfrequenz bzw. seinen Puls, seine aktuelle Atemfrequenz oder dergleichen überprüfen und gegebenenfalls aufgrund der angezeigten aktuellen Daten seine aktuelle körperliche Verfassung überprüfen bzw. überwachen.

Weiter ist es auch denkbar, dass mittels der Körperfunktionenerkennungseinrichtung, insbesondere nach positiver Körperfunktionserkennung, auch erkannt werden kann, in welchem Gesundheitszustand sich der Fahrzeugführer momentan befindet. Durch Abweichungen der aktuellen Herzfrequenz bzw. der aktuellen Atemfrequenz im Vergleich zu zuvor aufgenommenen Werten bzw. Sensordaten kann beispielsweise, je nach Abweichung, erkannt werden, dass der Fahrer müde ist oder wird bzw. dass der Fahrer gestresst ist. Weiter ist es auch denkbar, dass erkannt wird, dass ein Fahrer schwitzt bzw. dass dem Fahrer zu warm ist. Natürlich sind auch noch andere Möglichkeiten denkbar.

Vorteilhaft kann die erkannte Abweichung dem Fahrer ebenfalls angezeigt werden und entsprechend seiner aktuellen Gesundheitslage dem Fahrer eine Abhilfe angezeigt werden. Ist dem Fahrzeugführer beispielsweise zu warm, so dass er verstärkt schwitzt, so wird dies erkannt und dem Fahrer beispielsweise dies mitgeteilt mit dem Hinweis, dass die Klimaanlage aktiviert werden sollte, um dem verstärkten Schwitzen entgegenzuwirken. Ist der Fahrer hingegen gestresst, so erhöht sich sein Puls, also die Herzfrequenz, sowie die Atmungsfrequenz. Wird eine Stresssituation erkannt, so kann dem Fahrer beispielsweise mitgeteilt werden, das Fahrzeug abzustellen. Weiter kann vorzugsweise auch eine für den Fahrer lebensgefährdende Gesundheitslage festgestellt werden, beispielsweise ein Herzstillstand oder dergleichen. Sollte eine derartige Gesundheitslage festgestellt werden, so wird das Fahrzeug sowie der Fahrzeugsitz mit allen verfügbaren Funktionen vorzugsweise deaktiviert, so dass keine weiteren Personen verletzt oder Gegenstände beschädigt werden.

Insgesamt können Fahrzeugfunktionen und/oder Fahrzeugsitzfunktionen in Abhängigkeit der erfassten Sensordaten gesteuert werden. Vorteilhafterweise werden die Funktionen jedoch nicht automatisch aktiviert oder deaktiviert, sondern zunächst dem Fahrer mitgeteilt, beispielsweise auf einem Display oder dergleichen dargestellt, und erst nach einer Bestätigung des Fahrers aktiviert oder deaktiviert. Insbesondere bei einer positiven Sitzbelegungserkennung werden die Fahrzeugfunktionen für den Fahrzeugführer freigegeben, das heißt, die Fahrzeugfunktionen können durch Betätigung aktiviert oder deaktiviert werden.

Vorzugsweise wird der Verfahrensschritt einer Erfassung von Körperfunktionen erst nach dem Verfahrensschritt der Volumendetektion ausgeführt und besonders bevorzugt wird der Verfahrensschritt der Erfassung der Körperfunktionen erst nach einem positiven verlaufenden Verfahrensschritt der Volumendetektion ausgeführt. Dies bedeutet, dass zuerst ein Volumen detektiert werden muss, bevor entschieden wird, ob es sich um einen Gegenstand oder um ein Lebewesen, vorzugsweise einen Menschen, handelt.

Die Aufgabe der vorliegenden Erfindung wird ebenfalls gelöst durch eine Sitzbelegungserkennungseinrichtung, umfassend mindestens eine Volumendetektionseinrichtung mit einem ersten Sensor, welcher zur Detektion eines Volumens auf dem Fahrzeugsitz vorgesehen und ausgebildet ist, und mindestens eine Körperfunktionenerfassungseinrichtung mit einem zweiten Sensor, welcher zum Erfassen von Körperfunktionen vorgesehen und ausgebildet ist, insbesondere nach der Detektion eines Volumens. Erfindungsgemäß umfassen jeweils die Volumendetektionseinrichtung und die Körperfunktionenerfassungseinrichtung mindestens einen Sensor, mittels welchem ein Volumen bzw. Körperfunktionen erfassbar sind. Weiter bevorzugt weist die Sitzbelegungserkennungseinrichtung mindestens eine Körperfunktionserkennungseinrichtung zum Erkennen der erfassten Körperfunktionen.

Mittels der Volumendetektionseinrichtung kann kumulativ oder alternativ auch eine Masse erkannt werden, so dass eine Sitzbelegungserkennung erkannt wird, wenn sich eine Masse auf dem Fahrzeugsitz befindet. Weiter alternativ oder kumulativ können Umrisse erkannt werden, um so eine Sitzbelegung zu erkennen.

Denkbar sind weiterhin auch Sensoren bzw. Sensorvorrichtungen, welche gleichzeitig eine Sitzbelegung und eine Körperfunktion erfassen bzw. registrieren können, wobei natürlich durch derartige Sensoren bzw. Sensorvorrichtungen die aufgenommenen Signale richtig ausgelesen und verarbeitet werden müssen.

Gemäß einer bevorzugten Ausführungsform ist mittels des ersten Sensors der Volumendetektionseinrichtung zusätzlich mindestens eines aus einer Umrissdetektion und einer Massendetektion durchführbar.

Gemäß einer weiteren bevorzugten Ausführungsform ist mittels des zweiten Sensors der Körperfunktionserfassungseinrichtung mindestens eines aus Herzfunktion und Lungenfunktion erkennbar, insbesondere eines aus Herzfrequenz, Puls und Atmungsfrequenz.

Weiter vorteilhaft umfasst die Sitzbelegungserkennungseinrichtung eine Speichereinrichtung, mittels welcher die von der Volumendetektionseinrichtung und Körperfunktionenerfassungseinrichtung aufgenommenen Sensordaten abspeicherbar sind.

Erfindungsgemäß umfasst die Sitzbelegungserkennungseinrichtung eine Körperfunktionserkennungseinrichtung zum Erkennen von Körperfunktionen. So können die aufgenommenen Körperfunktionen vorteilhaft auch einem Menschen zugeordnet werden und dadurch eine Sitzbelegung durch ein Tier ausgeschlossen werden.

Gemäß einer weiteren bevorzugten Ausführungsform ist der zweite Sensor ausgebildet und vorgesehen, Sensordaten aufzunehmen und diese an die Körperfunktionserkennungseinrichtung zu übermitteln, wobei durch die Körperfunktionserkennungseinrichtung zum Bestimmen einer Gesundheitslage ein Vergleich der Sensordaten mit zuvor aufgenommenen Sensordaten durchführbar ist.

Durch einen Vergleich von Sensordaten mit zuvor aufgenommenen Sensordaten kann gegebenenfalls eine Abweichung von einem Soll-Wert oder eine Entwicklung der Werte beobachtet werden und aus der entsprechenden Beobachtung eine Schlussfolgerung gezogen werden. Beispielsweise kann aus einer kontinuierlichen Erhöhung der Körpertemperatur darauf geschlossen werden, dass beispielsweise dem Fahrer zu warm ist.

Weiter vorteilhaft umfasst die Sitzbelegungserkennungseinrichtung eine Steuereinrichtung, die vorzugsweise mit der Volumendetektionseinrichtung und der Körperfunktionenerfassungseinrichtung zumindest in signaltechnischer Verbindung steht. Mittels der Steuereinrichtung kann zuerst eine Volumendetektion mittels der Volumendetektionseinrichtung veranlasst werden und nach durchgeführter Volumendetektion eine Körperfunktionserfassung. Insbesondere kann der Ausgang der Volumendetektion durch die Steuereinrichtung weiter verwendet werden. Ist die Volumendetektion negativ, so ist beispielsweise keine Körperfunktionserfassung möglich, da die Grundvoraussetzung für eine Sitzbelegung nicht gegeben ist.

Ist die Volumendetektion jedoch positiv, so wird mittels der Steuereinrichtung eine Körperfunktionserfassung veranlasst.

Besonders vorteilhaft ist die Sitzbelegungserkennung aktiv, solange das Fahrzeug, insbesondere Fahrzeugfunktionen, aktiv sind oder aktivierbar sind.

Beispielsweise kann ein Fahrzeug als aktiv gelten, wenn zwar noch kein Motor gestartet wurde, aber bereits die Zündung aktiviert wurde, da mit Aktivierung der Zündung bereits viele Fahrzeugfunktionen zur Verfügung stehen.

Es ist denkbar, dass solange das Fahrzeug aktiv ist, ununterbrochen eine Sitzbelegungserkennung durchgeführt wird. Alternativ ist es auch denkbar, dass die Sitzbelegungserkennung nach regelmäßigen oder unregelmäßigen Zeitperioden durchgeführt wird. Die Zeitperioden sind hierbei natürlich entsprechend zu wählen und einzustellen. Ein zu langer Zeitraum könnte zu einer Umgehung der Sitzbelegungserkennung führen, was aufgrund von sicherheitstechnischen Angelegenheiten natürlich unerwünscht ist.

Weiter ist es vorstellbar, dass die Sitzbelegungserkennung dann durchgeführt wird, wenn ein bestimmtes Ereignis auftritt, die Sitzbelegungserkennung ist Ereignis-getriggert, etwa durch die Betätigung von Fahrzeugfunktionen.

Besonders bevorzugt sind die Volumendetektionseinrichtung und die Körperfunktionserfassungseinrichtung derart ausgestaltet, dass eine berührungslose Detektion bzw. Erfassung des entsprechenden Volumens und der entsprechenden Körperfunktionen möglich ist. Natürlich ist auch eine nicht-berührungslose Detektion bzw. Erfassung denkbar, jedoch ist es für einen Fahrzeugführer komfortabler, wenn eine berührungslose Detektion bzw. Erfassung möglich ist.

Besonders bevorzugt ist der erste Sensor und der zweite Sensor jeweils eines ausgewählt aus ein optischer, elektrischer, magnetischer, elektromagnetischer, thermischer, kapazitiver, akustischer oder mechanischer Sensor.

Weiter kann eine Körperfunktionserkennungseinrichtung vorgesehen sein, mittels welcher erkennbar ist, ob die aufgenommenen Sensordaten einem Menschen zuordenbar sind oder nicht. Somit kann ausgeschlossen werden, dass sich beispielsweise ein Tier auf dem Sitz befindet und die Sitzbelegungserkennung somit umgangen wird.

Weiter kann die Sitzbelegungserkennungseinrichtung einen Speicher bzw. eine Speichervorrichtung umfassen, mittels welcher die aufgenommenen Sensordaten abspeicherbar sind. Diese abgespeicherten Werte können dann für eine weitere Verwendung wieder abgerufen werden, beispielsweise um einen Verlauf der Herzfrequenz oder der Atemfrequenz darzustellen oder bestimmte Gesundheitslagen zu erkennen durch den Vergleich mit zuvor aufgenommenen Sensordaten.

Weiter bevorzugt handelt es sich bei den verwendeten Sensoren entweder um passive oder aktive Sensoren, wobei passive Sensoren bevorzugt werden, da die Sitzbelegungserkennung möglichst einfach ausgestaltet sein soll und auch nach Möglichkeit die Schaltung oder den Aufbau des Fahrzeugsitzes nicht unnötig verkomplizieren.

Weitere vorteilhafte Ausführungsformen ergeben sich aus den Unteransprüchen.

Weitere Ziele, Vorteile und Zweckmäßigkeiten der vorliegenden Erfindung sind der nachfolgenden von der Beschreibung in Verbindung mit der Zeichnung zu entnehmen. Hierbei zeigen:
- Fig. 1A: einen Fahrzeugsitz mit einem möglichen detektierbaren Volumen;
- Fig. 1B: den Fahrzeugsitz gemäß Fig. 1A in einer Draufsicht;
- Fig. 1C: den Fahrzeugsitz gemäß Fig. 1A in einer Vorderansicht;
- Fig. 1D: den Fahrzeugsitz gemäß Fig. 1A in einer Seitenansicht;
- Fig. 2: einen Fahrzeugsitz mit einem möglichen detektierbaren Volumen und einem zu detektierenden Volumen;
- Fig. 3: eine Sitzbelegungserkennungseinrichtung gemäß einer besonders bevorzugten Ausführungsform;
- Fig. 4: eine berührungslose Erfassung einer Körperfunktion;
- Fig. 5A-5D: ein Verfahren gemäß einer besonders bevorzugten Ausführungsform.

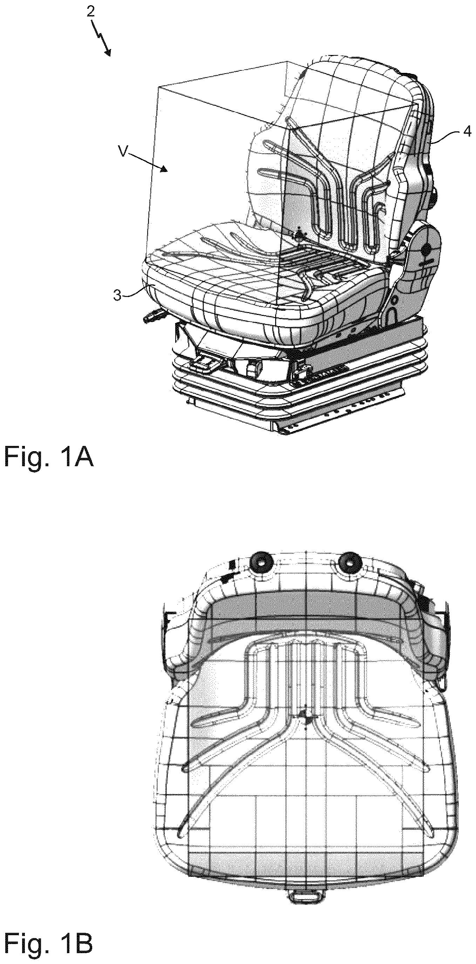

Figur 1A zeigt einen Fahrzeugsitz 2 und ein mögliches detektierbares Volumen V in einer perspektivischen Ansicht, in welchem mittels einer Volumendetektionseinrichtung 1 (hier nicht gezeigt) ein innerhalb des Volumens V befindliches Volumen V' detektierbar ist. Das Volumen V befindet sich wie gezeigt auf dem Fahrzeugsitz 2 und wird in etwa eingeschlossen durch ein Sitzteil 3 und einem Rückenlehnenteil 4. Natürlich sind auch andere Volumina denkbar, wobei ein derartiges Volumen V dahingehend sinnvoll ist, da sich innerhalb dieses Volumens V mit sehr großer Wahrscheinlichkeit ein Fahrzeugführer befindet, wenn er ein Fahrzeug betätigen möchte. Ein Volumen, welches eine Person oder ein Gegenstand einnimmt und in dem detektierbaren Volumen V sich befindet, kann daher detektiert werden.

Vorliegend ist aus den Figuren 1A bis 1D keine Sitzbelegungserkennungseinrichtung 5 erkennbar, da diese vorzugsweise in den Fahrzeugsitz 2 integriert ist, um dem Fahrzeugführer nicht im Fahrbetrieb oder allgemeiner im Sitzen nicht zu beeinflussen oder zu beeinträchtigen.

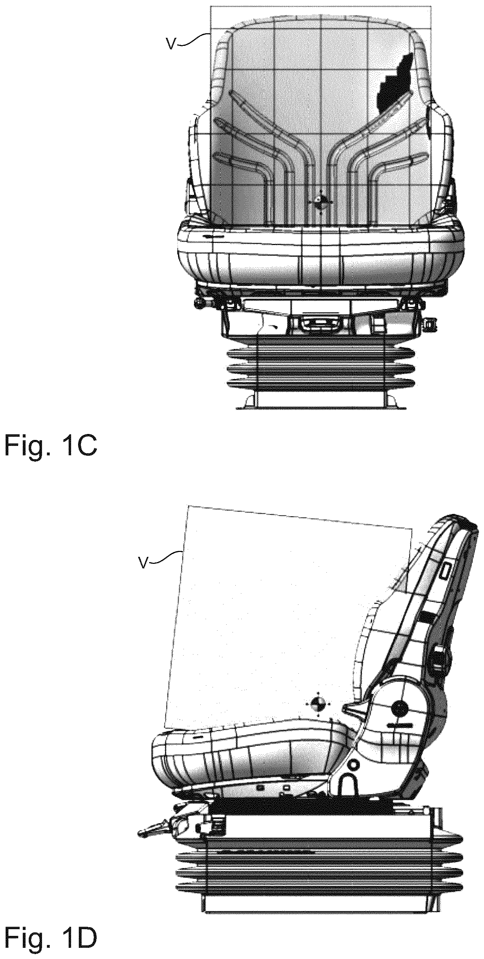

Figur 1B zeigt den Fahrzeugsitz gemäß Figur 1A in einer Draufsicht, Figur 1C in einer Vorderansicht und Figur 1D in einer Seitenansicht.

Natürlich sind auch noch andere Volumenbereiche V denkbar, da ein Mensch auch außerhalb des in den Figuren 1A-1D gezeigten Volumens V Volumenelemente V' aufweist oder aufweisen kann.

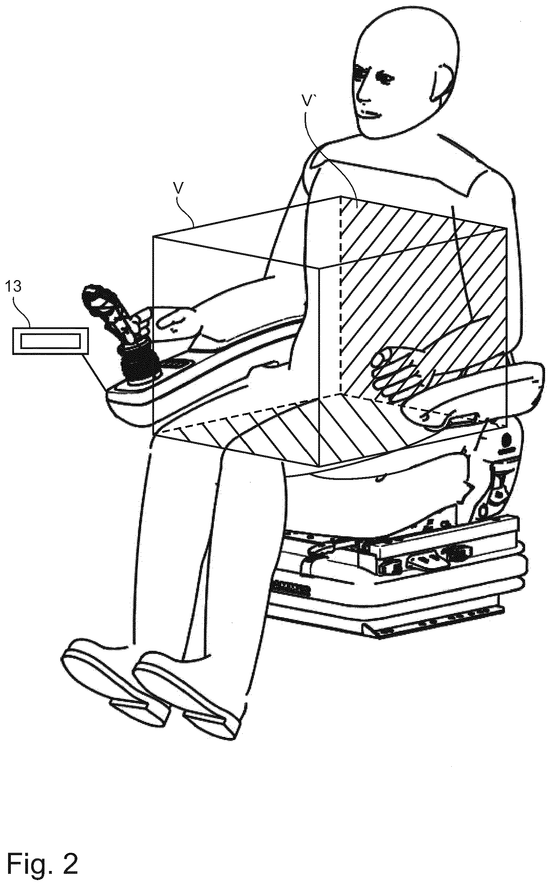

Figur 2 zeigt hierbei noch einmal das mögliche Volumen V, wobei bei einem auf dem Fahrzeugsitz 2 sitzende Person das Volumen V', dargestellt durch die schraffierte Fläche/das schraffierte Volumen, detektierbar ist.

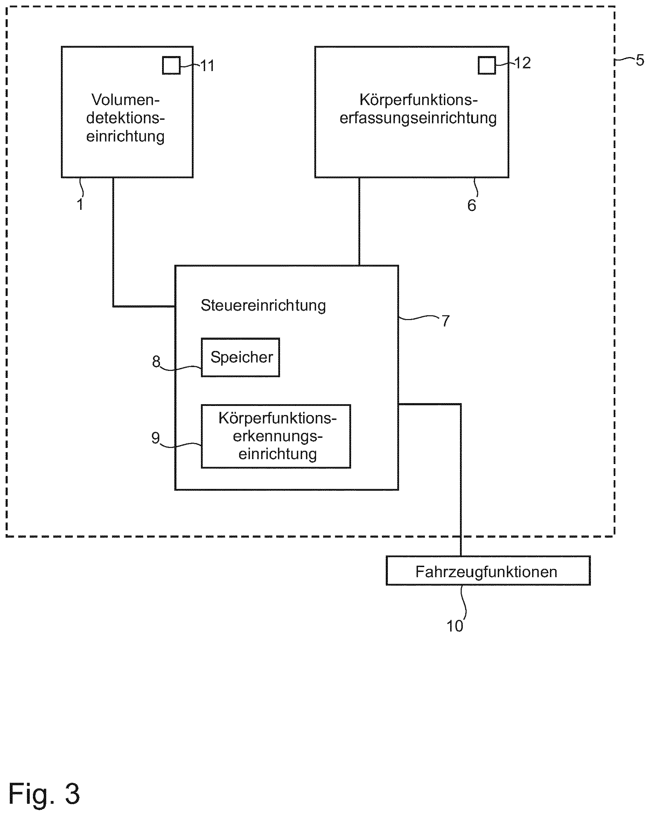

Die Figur 3 zeigt hier eine mögliche Ausgestaltung der Sitzbelegungserkennungseinrichtung 5, umfassend eine Volumendetektionseinrichtung 1 mit einem ersten Sensor 11, eine Körperfunktionserfassungseinrichtung 6 mit einem zweiten Sensor 12, wobei die Volumendetektionseinrichtung 1 und die Körperfunktionserfassungseinrichtung 6 jeweils mit einer Steuereinrichtung 7 zumindest signaltechnisch in Verbindung stehen. Die Steuereinrichtung 7 umfasst vorliegend eine Speichereinheit 8 und eine Erkennungseinrichtung 9 bzw. eine Körperfunktionserkennungseinrichtung 9.

Weiter ist vorteilhaft die Sitzbelegungserkennungseinrichtung 5, insbesondere die Steuereinrichtung 7, zumindest signaltechnisch mit den jeweiligen Fahrzeugfunktionen 10 in Verbindung, um die jeweiligen Fahrzeugfunktionen 10 freizugeben oder zu sperren, je nachdem, ob die Sitzbelegungserkennung positiv oder negativ ausgefallen ist.

Es ist auch denkbar, dass die Speichereinheit 8 und die Erkennungseinrichtung 9 nicht in der Steuereinrichtung 7 integriert sind, sondern eigenständige Einrichtungen sind, die zumindest in signaltechnischer Verbindung mit der Steuereinrichtung 7 stehen.

Vorteilhafterweise sind sowohl die Volumendetektionseinrichtung 1 als auch die Körperfunktionserfassungseinrichtung 6 derart ausgestaltet, dass sie berührungslos die entsprechenden Werte detektieren können. Insbesondere ist der entsprechende erste Sensor 11 ausgestaltet, eine berührungslose Detektion durchzuführen.

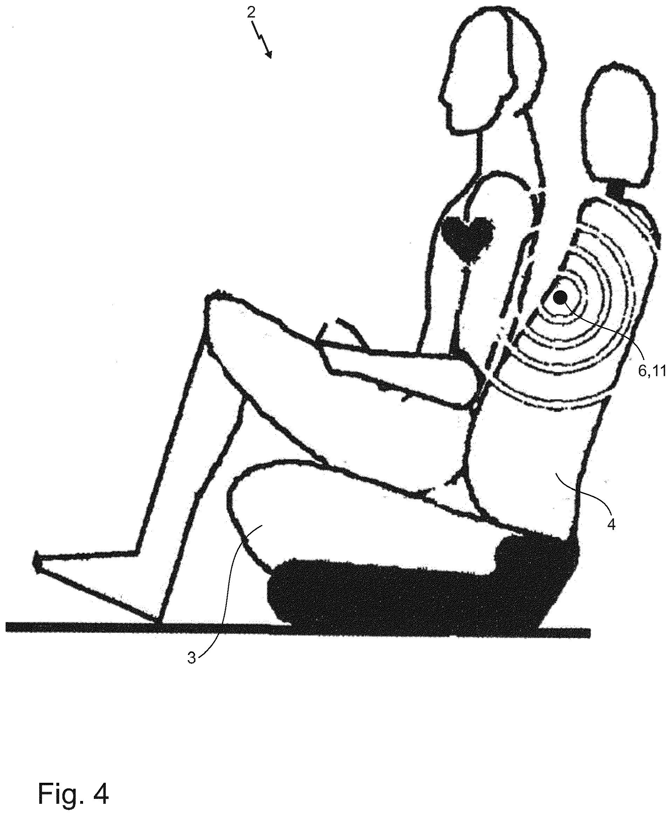

Die Figur 4 zeigt schematisch eine derartige berührungslose Erfassung einer Körperfunktion, vorliegend die Erfassung einer Herzfunktion, insbesondere einer Herzfrequenz. Wie zu erkennen ist, ist die Körperfunktionserfassungseinrichtung 6, insbesondere der zweite Sensor 12, in der Rückenlehne 4 integriert, wohingegen alternativ oder kumulativ eine Anordnung im Sitzteil 3 oder einem anderen beliebigen Element des Fahrzeugsitzes 2 oder auch des Fahrzeuges selbst integriert sein kann.

Es sind aus dem Stand der Technik verschiedene Möglichkeiten zur berührungslosen Detektion oder Erfassung eines Volumens bzw. einer Körperfunktion bekannt. Diese können elektrisch, elektronisch, kapazitiv, auf Ultraschall basierend, mechanisch, magnetisch, elektromagnetisch, akustisch, optisch, thermisch und dergleichen ausgebildet sein. Vorzugsweise werden kapazitive Methoden bevorzugt, da diese sehr einfach im Aufbau und sehr genau sind.

Vorzugsweise werden die Herzaktivität und/oder die Lungenaktivität der Person auf dem Fahrzeugsitz 2 durch den zweiten Sensor 12 erfasst. Weiter ist es denkbar, zusätzlich auch noch die Temperatur und/oder die Transpiration der Person zu erfassen, beispielsweise durch einen weiteren zweiten Sensor 12.

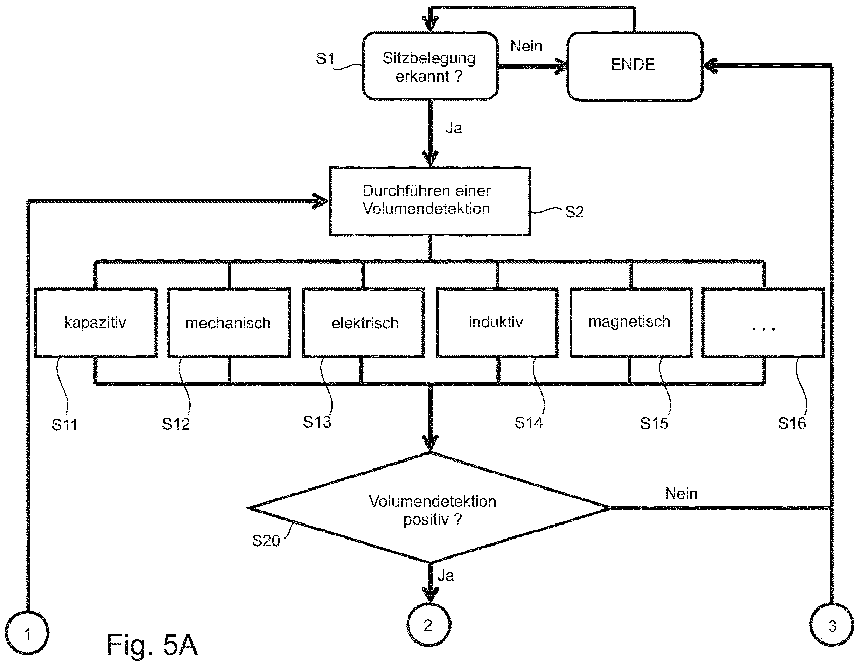

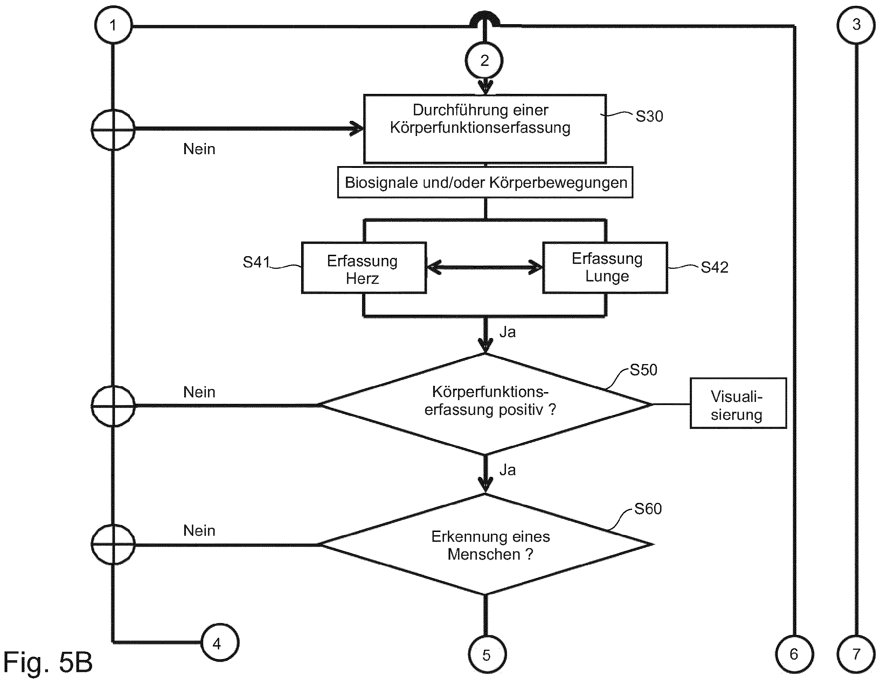

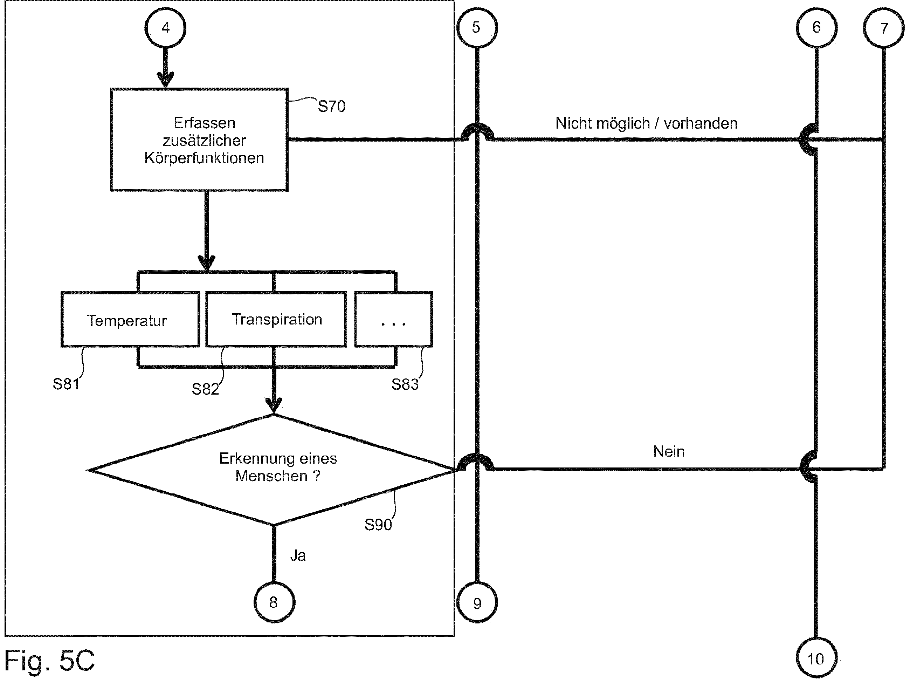

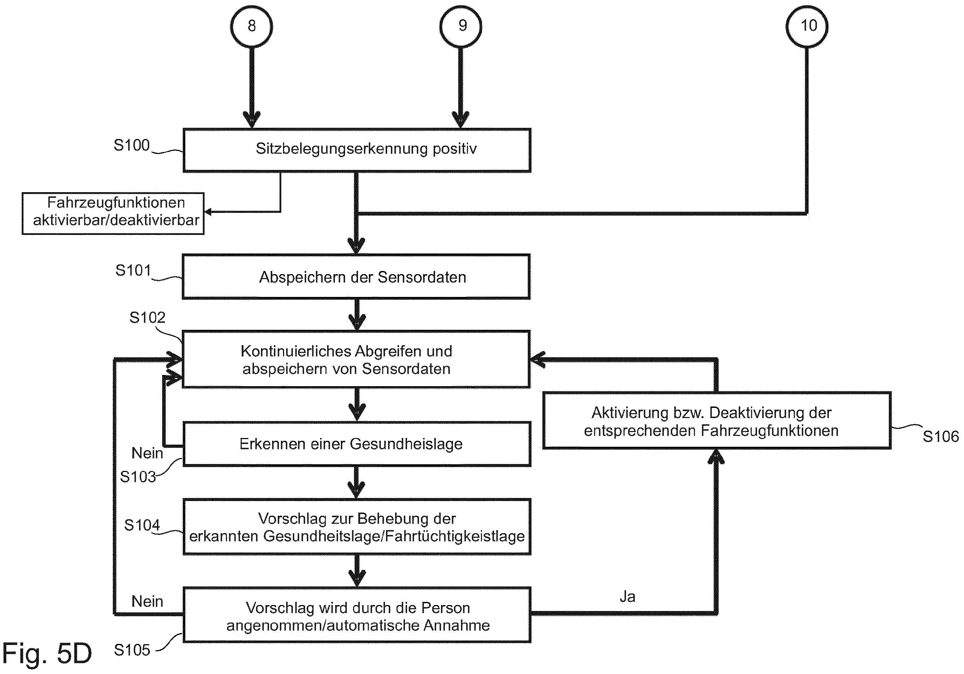

Ein möglicher Verfahrensablauf zur Sitzbelegungserkennung ist in den Figuren 5A-5D gezeigt und beschrieben.

Zunächst wird in einem Schritt S1 erkannt, vorzugsweise durch die Sitzbelegungserkennungseinrichtung 5, ob eine Sitzbelegungserkennung notwendig ist oder nicht. Ist sie nicht notwendig, so wird der Ablauf abgebrochen und gegebenenfalls neu gestartet. Eine Notwendigkeit kann hierbei sein, wenn das Fahrzeug aktiviert, gesteuert oder dergleichen werden soll. Es ist in diesen Fällen von besonderer Wichtigkeit, dass ein Fahrzeugführer sich bei diesen Aktivitäten auf dem Fahrzeugsitz 2 befindet, um die entsprechenden Bewegungen ausführen zu können. Erkannt werden kann dies durch entsprechende Sensoren und/oder einer Steuerung, dass ein Fahrzeugführer Fahrzeugfunktionen betätigt oder betätigen möchte.

Wird erkannt, dass eine Sitzbelegungserkennung durchgeführt werden muss, so wird mit dem Schritt S2 weiterverfahren, wobei der Schritt S2 der Durchführung einer Volumendetektion entspricht. Diese kann auf verschiedene Weisen durchgeführt werden, beispielsweise kapazitiv S11, mechanisch S12, elektrisch S13, induktiv S14, magnetisch S15 oder auf eine andere Art und Weise S16, wobei eine kapazitive Volumendetektion bevorzugt ist. Denkbar ist auch eine Detektion mittels Schallwellen oder eine optische Erkennung.

Wurde die Volumendetektion S2 durchgeführt mit einer oder einer Kombination aus S11 bis S16, so wird durch die Sitzbelegungserkennungseinrichtung 5 in einem weiteren Schritt S20 entschieden, ob ein Volumen detektiert wurde oder nicht. Fällt die Entscheidung negativ aus, so wird auch hier der Ablauf abgebrochen und gegebenenfalls neu gestartet. Eine Entscheidung basiert auf den Messwerten des ersten Sensors.

Ist hingegen die Volumendetektion S20 positiv, also befindet sich ein Volumen V' in dem Detektionsbereich V, so wird mittels der Sitzbelegungserkennungseinrichtung 5 eine Körperfunktionserfassung in einem Schritt S30 veranlasst. Die Körperfunktionserfassung kann hierbei durch eine Erfassung von Biosignalen und/oder von Bewegungen, insbesondere der Herzfunktionen/eines Pulses S41 und/oder einer Erfassung der Lungenfunktionen S42 durchgeführt werden. Natürlich ist es auch denkbar, anstelle der Herzfunktionen bzw. der Lungenfunktionen andere Körperfunktionen heranzuziehen, wobei bevorzugt die Herz- und Lungenfunktion abgegriffen werden sollte. Es ist hierbei auch denkbar, einerseits Biosignale, wie Herzfunktion oder Lungenfunktion, zu erkennen und/oder Körperbewegungen zu detektieren.

Nach Durchführung einer Erfassung der Herzfunktion S41 und/oder einer Erfassung der Lungenfunktion S42 wird anschließend in einem Schritt S50 das Ergebnis ausgewertet und geprüft. Ist die Körperfunktionserfassung negativ, so wird, abhängig von der Programmierung, entweder die Erfassung der Körperfunktionen S30 erneut durchgeführt oder der Ablauf wieder mit der Durchführung einer Volumendetektion S2 fortgeführt. Ist die Körperfunktionserfassung positiv verlaufen, so können die aufgenommenen Daten der Körperfunktionserfassung entsprechend visualisiert werden, beispielsweise mittels eines Displays oder dergleichen. Weiter können die Daten dokumentiert, also abgespeichert werden, um später ausgewertet oder im Falle eines Unfalls herangezogen werden.

Ist hingegen die Erfassung der Körperfunktionen positiv, also wurden die entsprechenden Körperfunktionen detektiert, so muss in einem Schritt S60 entschieden und erkannt werden, ob es sich hierbei um einen Menschen handelt oder um ein anderes Lebewesen, etwa um ein Tier, welches auf dem Fahrzeugsitz sitzt.

Wurde im Schritt S60 beispielsweise erkannt, dass es sich nicht um einen Menschen handelt, so können verschiedene Möglichkeiten der Weiterbehandlung herangezogen werden.

So ist es beispielsweise denkbar, die Körperfunktionserfassung S30 noch einmal zu starten und zu durchlaufen, um mögliche Fehler auszuschließen. Es ist alternativ denkbar, bereits bei dem Schritt S2 der Volumendetektion neu zu starten und die gesamte Sitzbelegungserkennung neu zu durchlaufen.

Es ist aber auch denkbar, weitere Körperfunktionen in einem Schritt S70 zu erfassen, beispielsweise eine Temperatur in einem Schritt S81, Transpiration in einem Schritt S82, wobei auch weitere Körperfunktionen S83 denkbar sind.

Ist es nicht möglich, weitere Körperfunktionen in einem Schritt S70 zu erfassen, so wird der Ablauf beendet und neu gestartet. Dies ist auch der Fall dafür, dass in einem Schritt S90 kein Mensch erkannt wird.

Wird jedoch in einem Schritt S90 erkannt, dass es sich dennoch um einen Menschen handelt, so ist die Sitzbelegungserkennung positiv S100. Wurde eine positive Sitzbelegung S100 erkannt, so wird direkt danach die Sitzbelegungserkennung neu gestartet, vorzugsweise mit dem Schritt der Volumendetektion S2, wobei natürlich auch andere Startpositionen denkbar sind, etwa ob eine Sitzbelegung erkannt wurde S1 oder die Durchführung der Körperfunktionserfassung S30. Durch eine positive Sitzbelegungserkennung ist es nun auch dem Fahrzeugführer möglich, entsprechende Fahrzeugfunktionen zu aktivieren bzw. zu deaktivieren. Hierzu zählen beispielsweise das Starten des Fahrzeugs und Steuerung von Sitzheizung, Lüftung, Klimaanlage etc.

Weiter wird, nachdem eine positive Sitzbelegungserkennung erkannt wurde, die entsprechend aufgenommenen Sensordaten in einem Schritt S101 abgespeichert, vorzugsweise in der Speichereinrichtung 8. Durch dieses Abspeichern können die Sensordaten zu späteren Zeitpunkten wieder aufgerufen werden und für andere Funktionen und Zwecke verwendet werden.

In einem weiteren Schritt S102 werden weiter Sensordaten kontinuierlich abgegriffen bzw. aufgenommen und natürlich ebenfalls abgespeichert. Dadurch ist es möglich, einen Verlauf von Körperfunktionen darzustellen und mit aktuell aufgenommenen Sensordaten zu vergleichen.

Durch diesen Vergleich kann zum Beispiel eine von der Normalen abweichenden Gesundheitslage bzw. Fahrtüchtigkeitslage erkannt werden. Steht beispielsweise der Fahrer unter Stress, so erhöht sich im Regelfall die Herzfrequenz und die Atmungsfrequenz. Eine Erhöhung dieser Frequenzen kann durch einen Vergleich mit vorherig aufgenommenen Sensordaten eben besonders leicht erkannt werden. Ebenso kann beispielsweise erkannt werden, dass die Herzfrequenz und die Atmungsfrequenz sich erniedrigen, was dann darauf hinweist, dass die Person auf dem Fahrzeugsitz müde wird. Wird eine derartige Gesundheitslage bzw. Fahrtüchtigkeitslage nicht erkannt, so werden weiterhin Sensordaten aufgenommen und weiterhin eine Erkennung der Gesundheitslage bzw. Fahrtüchtigkeitslage durchgeführt.

Durch Erkennen einer Gesundheitslage bzw. Fahrtüchtigkeitslage kann auch auf die Fahrtüchtigkeit des Fahrzeugführers geschlossen werden. Sollte die Gesundheitslage dramatisch gegenüber einem Normalzustand abweichen, so kann dem Fahrzeugführer die Fahrtüchtigkeit aberkannt werden und durch die Sitzbelegungserkennung die Fahrzeugfunktionen deaktiviert werden, das heißt, der Fahrzeugführer kann keine der Fahrzeugfunktionen mehr betätigen.

Eine Gesundheitslage bzw. Fahrtüchtigkeitslage kann insbesondere durch einen Vergleich von aktuellen Daten mit zuvor aufgenommenen Daten ermittelt werden. Insbesondere kann durch einen Vergleich der aktuellen Daten mit zuvor aufgenommenen Daten auch eine Tendenz ermittelt werden, beispielsweise ob der Fahrer müde wird oder dergleichen. Vorteilhaft wird ein Vergleich mit einem aufgenommenen oder einem zuvor abgespeicherten Anfangswert durchgeführt, es wird daher eine Entwicklung bzw. ein Vergleich mit der Gesundheitslage des Fahrers zu Beginn der Tätigkeit ermittelt.

Vorteilhaft kann nach Erkennen der abweichenden Gesundheitslage bzw. Fahrtüchtigkeitslage ein Vorschlag zur Behebung durch die Sitzbelegungserkennungseinrichtung 5 übermittelt werden, welcher der Person visuell und/oder akustisch mitgeteilt wird, etwa über ein Display angezeigt wird oder mittels einer gesprochenen Warnung angezeigt wird. Schwitzt beispielsweise eine Person sehr stark, so kann die Sitzbelegungserkennungseinrichtung 5 der Person vorschlagen, die Klimaanlage zu aktivieren. Es ist dabei besonders vorteilhaft, dass die Sitzbelegungserkennungseinrichtung 5 nicht automatisch die Klimaanlage aktiviert, sondern vorerst nur den Vorschlag der Person mitteilt. Die Person muss dann entweder den Vorschlag ablehnen oder annehmen. Falls die Person den Vorschlag ablehnt, so wird der Vorschlag nicht ausgeführt, wird der Vorschlag hingegen angenommen, so wird der Vorschlag ausgeführt, also beispielsweise die Klimaanlage aktiviert. Eine derartige Entscheidung wird in einem Schritt S105 durchgeführt. Es ist jedoch auch denkbar, dass eine automatische Aktivierung oder Deaktivierung von Fahrzeugfunktionen durch die Sitzbelegungserkennungseinrichtung 5 vorgenommen wird und der Fahrzeugführer eine visuelle oder akustische Mitteilung darüber erhält, beispielsweise eine Anzeige über ein Display oder ein Signalton.

Alternativ ist es auch denkbar, dass sowohl die Volumendetektion als auch die Körperfunktionserfassung gleichzeitig ausgeführt werden und nicht wie oben beschrieben hintereinander. Es ergeben sich hieraus dann vier verschiedene Kombinationen: Volumendetektion und Körperfunktionserfassung positiv, Volumendetektion und Körperfunktionserfassung negativ, Volumendetektion negativ und Körperfunktionserfassung positiv, Volumendetektion positiv und Körperfunktionserfassung negativ.

Sind Volumendetektion und Körperfunktionserfassung positiv, so wird darauf geschlossen, dass sich ein Lebewesen auf dem Fahrzeugsitz befindet. Vorzugsweise wird noch mittels einer Erkennungseinrichtung 9 erkannt, ob es sich bei dem Lebewesen um einen Menschen handelt oder nicht.

Sind Volumendetektion und Körperfunktionserfassung negativ, so wird darauf geschlossen, dass der Fahrzeugsitz unbelegt ist.

Ist die Volumendetektion negativ und die Körperfunktionserfassung positiv, so ist davon auszugehen, dass sich ein Mensch in der Nähe der Körperfunktionserfassungseinrichtung 6 befindet, jedoch nicht auf dem Fahrzeugsitz 2 selbst sitzt. Beispielsweise kann sich die Person hinter dem Fahrzeugsitz 2 befinden.

Ist die Volumendetektion positiv und die Körperfunktionserfassung negativ, so ist davon auszugehen, dass sich ein nicht-lebendiger Gegenstand auf dem Fahrzeugsitz befindet.

Eine positive Sitzbelegungserkennung ist hierbei auch nur dann gegeben, wenn die Volumendetektion und die Körperfunktionserfassung positiv ausgefallen sind.

Alternativ ist es auch denkbar, zuerst eine Körperfunktionserfassung durchzuführen und nach der Körperfunktionserfassung eine Volumendetektion durchzuführen.

Eine positive Sitzbelegungserkennung ist hierbei ebenfalls auch nur dann gegeben, wenn die Volumendetektion und die Körperfunktionserfassung positiv ausgefallen sind.

### Bezugszeichenliste

- 1: Volumendetektionseinrichtung
- 2: Fahrzeugsitz
- 3: Sitzteil
- 4: Rückenlehnenteil
- 5: Sitzbelegungserkennungseinrichtung
- 6: Körperfunktionserfassungseinrichtung
- 7: Steuereinrichtung
- 8: Speichereinrichtung
- 9: Körperfunktionserkennungseinrichtung
- 10: Fahrzeugfunktion
- 11: erster Sensor
- 12: zweiter Sensor
- 13: Display
- V, V': Volumen

## Patentansprüche

1. Verfahren zum Erkennen einer Sitzbelegung eines Sitzes,
**gekennzeichnet durch**
die Verfahrensschritte:
a. Durchführen einer Volumendetektion zur Detektion eines Volumens auf dem Fahrzeugsitz mittels einer Volumendetektionseinrichtung (1);
b. Durchführen einer Körperfunktionserfassung mittels einer Körperfunktionserfassungseinrichtung (6) nach positiver Volumendetektion;
c. Durchführen einer Körperfunktionserkennung mittels einer Körperfunktionserkennungseinrichtung (9) nach positiver Körperfunktionserfassung.

2. Verfahren nach Anspruch 1,
**dadurch gekennzeichnet, dass**
der Verfahrensschritt c zumindest eines aus Erfassen einer Herzfunktion und Erfassen einer Lungenfunktion umfasst.

3. Verfahren nach Anspruch 2,
**dadurch gekennzeichnet, dass**
nach einer negativen Körperfunktionserkennung entweder
- die Körperfunktionserfassung erneut durchgeführt wird, oder
- mit Verfahrensschritt a. fortgefahren wird, oder
- zumindest eine zusätzliche Körperfunktion durch die Körperfunktionserfassungseinrichtung (6) erfasst werden.

4. Verfahren nach Anspruch 3,
**dadurch gekennzeichnet, dass**
die mindestens eine zusätzliche Körperfunktion ausgewählt ist aus einer Gruppe, die Erfassen einer Temperatur, einer Transpiration, einer Magenakustik, Körpergeruch und Hirnwellen und/oder einer Kombination daraus umfasst.

5. Verfahren nach einem der vorangehenden Ansprüche,
**dadurch gekennzeichnet, dass**
Daten, welche von der Körperfunktionserfassungseinrichtung (6) aufgenommen werden, mittels eines Displays (13) angezeigt werden.

6. Verfahren nach Anspruch 2,
**dadurch gekennzeichnet, dass**
mittels der Körperfunktionserkennungseinrichtung (6) nach positiver Körperfunktionserkennung ein Gesundheitszustand erkannt wird.

7. Sitzbelegungserkennungseinrichtung (5) zum Erkennen einer Sitzbelegung eines Sitzes (2),
**gekennzeichnet durch**
mindestens eine Volumendetektionseinrichtung (1) mit einem ersten Sensor (11), welcher zur Detektion eines Volumens (V, V') auf dem Fahrzeugsitz (2) vorgesehen und ausgebildet ist, und mindestens eine Körperfunktionserfassungseinrichtung (6) mit einem zweiten Sensor (12), welcher zum Erfassen von Körperfunktionen vorgesehen und ausgebildet ist, und mindestens eine Körperfunktionserkennungseinrichtung (9) zum Erkennen der erfassten Körperfunktionen, wobei der zweite Sensor (12) ausgebildet und vorgesehen ist, Sensordaten aufzunehmen und diese an die Körperfunktionserkennungseinrichtung (9) zu übermitteln, wobei **durch** die Körperfunktionserkennungseinrichtung (9) zum Bestimmen einer Gesundheitslage ein Vergleich der Sensordaten mit zuvor aufgenommenen Sensordaten durchführbar ist.

8. Sitzbelegungserkennungseinrichtung (5) nach Anspruch 7,
**dadurch kennzeichnet, dass**
mittels des ersten Sensors (11) der Volumendetektionseinrichtung (1) zusätzlich mindestens eines aus einer Umrissdetektion und einer Massendetektion durchführbar ist.

9. Sitzbelegungserkennungseinrichtung (5) nach Anspruch 7 oder 8,
**dadurch gekennzeichnet, dass**
mittels des zweiten Sensors (12) der Körperfunktionserfassungseinrichtung (6) mindestens eines aus Herzfunktion und Lungenfunktion erkennbar ist.

10. Sitzbelegungserkennungseinrichtung (5) nach einem der Ansprüche 7-9,
**dadurch gekennzeichnet, dass**
die Sitzbelegungserkennungseinrichtung (5) eine Speichereinrichtung (8) aufweist, mittels welcher Sensordaten, welche von der Körperfunktionserfassungseinrichtung (6) aufnehmbar sind, abspeicherbar sind.

11. Sitzbelegungserkennungseinrichtung (5) nach der Ansprüche 7-10,
**dadurch gekennzeichnet, dass**
der erste Sensor (11) und der zweite Sensor (12) jeweils ausgewählt ist aus einer Gruppe, die einen optischen, elektrischen, magnetischen, elektromagnetischen, thermischen, kapazitiven, akustischen oder mechanischer Sensor umfasst.

12. Fahrzeugsitz (2) mit einer Sitzbelegungserkennungseinrichtung (5) nach einem der Ansprüche 7-11.

## Claims

1. Method for recognizing the seat occupancy of a seat,
**characterized by**
the method steps:
a. Performing a volume detection process for detecting a volume on a vehicle seat by means of a volume detection device (1),
b. Performing a body function detection process by means of a body function detection device (6) after positive volume detection,
c. Performing a body function recognition process by means of a body function recognition device (9) after positive body function detection.

2. Method according to claim 1,
**characterized in that**
the method step c. comprises at least one of detecting a cardiac function and detecting a pulmonary function.

3. Method according to claim 2,
**characterized in that**
after a negative body function recognition either
- the body function detection process is performed again, or
- one continues with method step a., or
- at least one additional body function is detected by the body function detection device (6).

4. Method according to claim 3,
**characterized in that**
the at least one additional body function is selected from a group which comprises detecting a temperature, perspiration, gastric acoustics, body odor and brain waves and/or a combination of these.

5. Method according to one of the preceding claims,
**characterized in that**
data that is recorded by the body function detection device (6) is shown by means of a display (13).

6. Method according to claim 2,
**characterized in that**
a health status is detected by means of the body function recognition device (6) after positive body function recognition.

7. Seat occupancy recognition device (5) for recognizing seat occupancy of a seat (2),
**characterized by**
at least one volume detection device (1) having a first sensor (11), which is provided and designed for detecting a volume (V, V') on the vehicle seat (2), and at least one body function detection device (6) having a second sensor (12), which is provided and designed for detecting body functions, and at least one body function recognition device (9) for recognizing the detected body functions, wherein the second sensor (12) is designed and provided to record sensor data and transmit these to the body function recognition device (9), wherein a comparison of the sensor data against previously recorded sensor data can be performed by means of the body function recognition device (9) to determine a health status.

8. Seat occupancy recognition device (5) according to claim 7,
**characterized in that**
by means of the first sensor (11) of the volume detection device (1) at least one of contour detection and mass detection can be performed additionally.

9. Seat occupancy recognition device (5) according to claim 7 or 8,
**characterized in that**
by means of the second sensor (12) of the body function detection device (6), at least one of cardiac function and pulmonary function can be detected.

10. Seat occupancy recognition device (5) according to one of the claims 7-9,
**characterized in that**
the seat occupancy recognition device (5) has a storage device (8) by means of which the sensor data, which can be recorded by the body function detection device (6), can be stored.

11. Seat occupancy recognition device (5) according to claims 7-10,
**characterized in that**
the first sensor (11) and the second sensor (12) are each selected from a group that comprises an optical, electrical, magnetic, electromagnetic, thermal, capacitative, acoustic or mechanical sensor.

12. Vehicle seat (2) having a seat occupancy recognition device (5) according to one of the claims 7-11.

## Revendications

1. Procédé de reconnaissance de l'occupation d'un siège,
**caractérisé par** les étapes de procédé :
a. réalisation d'une détection de volume pour la détection d'un volume sur le siège de véhicule au moyen d'un dispositif (1) de détection de volume ;
b. réalisation d'une détection de fonction corporelle au moyen d'un dispositif (6) de détection de fonction corporelle après détection de volume positive ;
c. réalisation d'une reconnaissance de fonction corporelle au moyen d'un dispositif (9) de reconnaissance de fonction corporelle après reconnaissance de fonction corporelle positive.

2. Procédé selon la revendication 1,
**caractérisé par le fait que**
l'étape de procédé c comporte au moins l'une de la détection d'une fonction cardiaque et de la détection d'une fonction pulmonaire.

3. Procédé selon la revendication 2,
**caractérisé par le fait qu'**
après une reconnaissance de fonction corporelle négative
- ou bien la reconnaissance de fonction corporelle est réalisée de nouveau ;
- ou bien on poursuit avec l'étape de procédé a ;
- ou bien au moins une fonction corporelle supplémentaire est détectée par le dispositif (6) de détection de fonction corporelle.

4. Procédé selon la revendication 3,
**caractérisé par le fait que**
ladite au moins une fonction corporelle supplémentaire est choisie dans un groupe qui comporte la détection d'une température, d'une transpiration, d'une acoustique d'estomac, une odeur corporelle et des ondes cérébrales et/ou une combinaison de ces fonctions.

5. Procédé selon l'une des revendications précédentes,
**caractérisé par le fait que**
des données, qui sont reçues par le dispositif (6) de détection de fonction corporelle, sont affichées au moyen d'un écran de visualisation (13).

6. Procédé selon la revendication 2,
**caractérisé par le fait qu'**
un état de santé est reconnu au moyen du dispositif (6) de reconnaissance de fonction corporelle après reconnaissance de fonction corporelle positive.

7. Dispositif (5) de reconnaissance d'occupation de siège pour la reconnaissance de l'occupation d'un siège (2),
**caractérisé par**
au moins un dispositif (1) de détection de volume comportant un premier capteur (11), lequel est prévu et conçu pour la détection d'un volume (V, V') sur le siège de véhicule (2), et au moins un dispositif (6) de détection de fonction corporelle comportant un second capteur (12), lequel est prévu et conçu pour la détection de fonctions corporelles, et au moins un dispositif (9) de reconnaissance de fonction corporelle pour la reconnaissance des fonctions corporelles détectées, le second capteur (12) étant conçu et prévu pour recevoir des données de capteur et pour transmettre celles-ci au dispositif (9) de reconnaissance de fonction corporelle, une comparaison des données de capteur à des données de capteur précédemment reçues étant réalisable par le dispositif (9) de reconnaissance de fonction corporelle pour la détermination d'une situation de santé.

8. Dispositif (5) de reconnaissance d'occupation de siège selon la revendication 7,
**caractérisé par le fait qu'**
en outre au moins l'une d'une détection de contour et d'une détection de masse est réalisable au moyen du premier capteur (11) du dispositif (1) de détection de volume.

9. Dispositif (5) de reconnaissance d'occupation de siège selon l'une des revendications 7 ou 8,
**caractérisé par le fait qu'**
au moins l'une de la fonction cardiaque et de la fonction pulmonaire est détectable au moyen du second capteur (12) du dispositif (6) de détection de fonction corporelle.

10. Dispositif (5) de reconnaissance d'occupation de siège selon l'une des revendications 7 à 9,
**caractérisé par le fait que**
le dispositif (5) de reconnaissance d'occupation de siège présente un dispositif de mémorisation (8), au moyen duquel des données de capteur, lesquelles peuvent être reçues par le dispositif (6) de détection de fonction corporelle, sont mémorisables.

11. Dispositif de reconnaissance d'occupation de siège (5) selon l'une des revendications 7 à 10,
**caractérisé par** la fait que
le premier capteur (11) et le second capteur (12) sont chacun choisis dans un groupe qui comporte un capteur optique, électrique, magnétique, électromagnétique, thermique, capacitif, acoustique ou mécanique.

12. Siège de véhicule (2) comportant un dispositif (5) de reconnaissance d'occupation de siège selon l'une des revendications 7 à 11.
